# EUROPEAN PATENT APPLICATION

(11) **EP 1 908 354 A1**
(43) Date of publication of application: **09.04.2008**
(21) Application number: 06781534.0
(22) Date of filing: 25.07.2006
(51) Int. Cl.: A23C 9/127, A23L 1/29, A23L 1/305, A61K 38/00, A61P 9/12, C12N 9/48, C12P 21/02

(54) **PROCESS FOR PRODUCTION OF FERMENTED MILK AND FERMENTED MILK BEVERAGE/FOOD**

(30) Priority: 26.07.2005 JP 2005216037
(71) Applicant: Calpis Co., Ltd., Shibuya-ku, Tokyo 150-0022 (JP)
(72) Inventor: YASUDA, Mariko c/o CALPIS CO., LTD. Food & Drink, Sagamihara-shi, Kanagawa 2290006 (JP); SHINODA, Tadashi c/o CALPIS CO., LTD. Functional, Fuchinobe Sagamihara-shi, Kanagawa 22900 (JP); YAMAMOTO, Naoyuki c/o CALPIS CO., LTD. Functional, Fuchinobe, Sagamihara-shi, Kanagawa 2290 (JP)
(74) Representative: Hallybone, Huw George
(86) International application number: PCT/JP2006/314622
(87) International publication number: WO 2007/013426

(57) **Abstract**

The invention provides a method for producing a fermented milk that conveniently gives excellent taste and flavor orfunctionalities, such as antihypertensive effect, to fermented food and beverages. The method enables effective production of particular peptides having useful functions, such as antihypertensive effect, in the resulting fermented milk. The invention also provides fermented milk food and beverages utilizing the fermented milk, which have excellent taste and flavor and even functionality. The present method includes the steps of (A) enzymatically digesting animal milk casein with protease (1)containing particular proteases such as papain, and (B) fermenting animal milk casein or the like with lactic acid bacteria, to thereby produce VPP, IPP, and YP in the resulting fermented milk. The fermented milk food and beverages of the present invention contain the fermented milk obtained by the present method or a concentrate thereof, and VPP, IPP, and YP at or over particular contents.

## Description

The present invention relates to a method for producing a fermented milk, which facilitates effective production of peptides having confirmed useful functions, e.g., an antihypertensive effect, such as Val-Pro-Pro, Ile-Pro-Pro, and Tyr-Pro, in the resulting fermented milk, and which conveniently gives excellent taste and flavor to the resulting fermented milk that is useful for producing fermented milk food and beverages. The present invention also relates to fermented milk food and beverages utilizing the fermented milk obtained by this method.

Peptides having useful functions, such as an antihypertensive effect, have been isolated from enzymatic digestion products of food protein or fermented food, and reported. In particular, peptides having a residue Pro at their carboxyl terminal often have so called angiotensin converting enzyme (ACE) inhibitory activity, which contributes to regulation of blood pressure in living organisms. Such peptides have been demonstrated in vivo to exhibit an antihypertensive effect. Non-patent Publications 1 and 2 reported that, among various antihypertensive peptides, Val-Pro-Pro, Ile-Pro-Pro, and Tyr-Pro, which are hydrolysate peptides of milk protein casein, had particularly strong antihypertensive activity in spontaneously hypertensive rats.

A method for producing such functional peptides was reported, for example, in Patent Publication 1, wherein the functional peptides were produced by fermenting milk components with lactic acid bacteria, such as *Lactobacillus helveticus.* Patent Publications 2 to 4 proposed methods for producing the functional peptides by enzymatic digestion of casein, the primary milk protein.

The methods employing the enzymatic digestion disclosed in Patent Publications 2 to 4 allow effective production of the functional peptides under suitably selected conditions, such as the amount of enzyme to be used.

However, the functional peptides obtained by such enzymatic digestion need to undergo purification, concentration, powdering, and other processes, prior to use in production of processed milk products having a high functional peptide content as well as good taste and flavor, such as fermented milk food. This inevitably complicates the production process.

On the other hand, the methods employing the lactic acid bacteria fermentation allow direct production of a fermented milk that may be used for food, beverages, or starting materials thereof, and the functional peptides are produced in the fermented milk. Thus the functional peptides do not necessarily undergo purification, concentration, powdering, and other processes, and the obtained fermented milk may be used conveniently in production of processed milk products.

However, the fermented milk directly obtained by the method employing the lactic acid bacteria fermentation disclosed in Patent Publication 1, contained a substantial amount of undigested casein, and the content of the functional peptides therein was low. Thus a method has been demanded that could further improve the production efficiency of the functional peptides.

Patent Publication 5 proposed particular lactic acid bacteria that improve productivity of the functional peptides having antihypertensive activity and other properties, and that hydrolyze casein more effectively, as well as fermented milk produced with such lactic acid bacteria.

Non-patent Publication 3 reported that the effective amount of Val-Pro-Pro and Ile-Pro-Pro as active components for achieving antihypertensive effect was 3.4 mg or more per day in adults in terms of the total amount of these peptides. In order to produce an antihypertensive final product containing the effective amount of the peptides from, for example, the fermented milk prepared with the lactic acid bacteria disclose in the above Patent Publication 5, not less than 60 ml of the fermented milk is required.

On the other hand, technical development has recently been demanded for further improvement of taste and flavor of fermented milk food and beverages. In light of this, a method for producing a fermented milk is demanded that further improves the content of the functional peptides produced in a lactic acid bacteria fermented milk. Such a method is desired for facilitating development of recipes for fermented milk food and beverages, and for producing functional food materials with still better taste and flavor or fermentedmilk food and beverages with an increased amount of the functional peptides.
Patent Publication 1: JP-2782142-B
Patent Publication 2: JP-6-128287-A
Patent Publication 3: JP-2001-136995-A
Patent Publication 4: WO-2005-012542-A
Patent Publication 5: JP-3028411-B
Non-patent Publication 1: Nakamura et al., J. Dairy Sci. 78: p1253-1257 (1995)
Non-patent Publication 2: Yamamoto et al., J. Dairy Sci. 82: p1388-1393 (1995)
Non-patent Publication 3: Hata et al. , Am. J. Clin. Nutr., p767-771 (1996)

It is an object of the present invention to provide a method for producing a fermented milk that conveniently gives excellent taste and flavor or functionalities, such as antihypertensive effect, to fermented milk food and beverages, which method enables effective production of particular peptides having useful functions, such as antihypertensive effect, in the resulting fermented milk.

It is another object of the present invention to provide fermented milk food and beverages utilizing the above fermented milk, which have excellent taste and flavor and even functionality.

According to the present invention, there is provided a method for producing a fermented milk comprising the steps of:
(A) enzymatically digesting animal milk casein with protease (1) comprising at least one of papain, bromelain, and proteases belonging to a superfamily that shows enzyme reaction similar to these, and
(B) fermenting, with lactic acid bacteria, animal milk casein and/or protease (1) -digestion product of animal milk casein,
to thereby produce at least one of Val-Pro-Pro (sometimes referred to as VPP hereinbelow), Ile-Pro-Pro (sometimes referred to as IPP hereinbelow), and Tyr-Pro (sometimes referred to as YP hereinbelow) in a resulting fermented milk.

According to the present invention, there is also provided fermented milk food and beverages comprising a fermented milk obtained by the above method or a concentrate thereof, wherein an animal milk having a casein content of 3 to 10 wt% is used as said animal milk casein, and an amount of said protease (1) added is 0.005 to 0.1 wt% of the amount of said casein,
said fermented milk food and beverages containing not less than 1.2 mg/100 ml of Val-Pro-Pro, not less than 1.0 mg/100 ml of Ile-Pro-Pro, and not less than 0.5 mg/100 ml of Tyr-Pro.

In the method for producing a fermented milk according to the present invention, since steps (A) and (B) discussed above are performed in combination, the particular peptides having useful functions, such as antihypertensive effect, may efficiently be produced in the resulting fermented milk. In addition, good taste and flavor as well as functionalities, such as antihypertensive effect, may conveniently be given to the fermented milk food and beverages. In particular, by carrying out steps (A) and (B) simultaneously, the production process may be simplified, and the production efficiency of the particular peptides may be improved.

Since the fermented milk food and beverages according to the present invention utilize the fermentedmilk obtained by the present method, which contains a substantial amount of the particular peptides having useful functions, their taste and flavor may be controlled easily, and excellent taste and flavor as well as functionality may be given to the products.

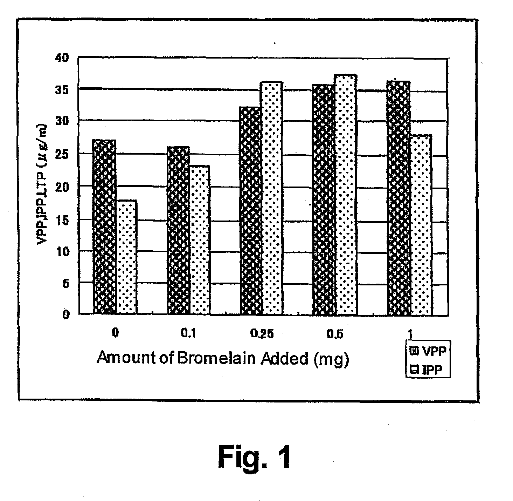

Fig. 1 is a graph showing the amounts of VPP and IPP peptides produced at different amounts of bromelain added in Example 4.

The present invention will now be explained in detail.

The method according to the present invention includes step (A) of enzymatic digestion of animal milk casein with particular protease (1).

The particular protease (1) contains at least one of papain, bromelain, and proteases belonging to a superfamily that shows enzyme reaction similar to these. The protease (1) contains proteases that have specificity to β-casein, which has a peptide sequence, VPP, IPP, YP, or the like, and complement and reinforce protease activity that otherwise appears to be insufficient in the initial digestion process of animal milk casein in ordinary lactic acid bacteria fermentation, to thereby improve the productivity of the peptides with desired functionality.

The papain, bromelain, and proteases belonging to a superfamily that shows enzyme reaction similar to these usually include a protease that cleaves at least one peptide represented by (Xaa)m-Val-Pro-Pro-(Xbb)n, (Xaa)m-Ile-Pro-Pro-(Xbb)n, or (Xaa)m-Tyr-Pro-(Xbb)n at the carboxyl terminal side of (Xaa)m, wherein Xaa and Xbb each independently stands for an arbitrary amino acid; Xaa in one peptide sequence may be the same as or different from Xaa in the other peptide sequences; Xbb in one peptide sequence may be the same as or different from Xbb in the other peptide sequences; m and n are integers; when m is 2 or more, Xaa's may be the same or different; and when n is 2 or more, Xbb's may be the same or different.

The papain may be a papaya extract, and the bromelain may be a pineapple extract.

Examples of the proteases belonging to the above-mentioned superfamily may include plant cysteine peptidases that have a cysteine residue at the active center like papain, such as ananain, ananase, extranase, pinase, pineapple enzyme, traumanase, papayotin, summetrin, velardon, papaya peptidase, chymopapain, papaya proteinase, PPIV, caricain, proteinase omega, and enzymes belonging to the peptidase family C1, with proteases belonging to a subfamily of papain being preferred. Further, cathepsin may also be included, of which active center is extremely homologous to that of these enzymes, so that it is believed to have substrate specificity similar to these enzymes.

Protease (1) to be used in step (A) may be a mixture of a plurality of enzymes, or may optionally contain a particular protease other than the above for improving digestivity.

Commercial products may be used as protease (1), such as Papain F (trade name, manufactured by AMANO ENZYME INC.), VERON L10 (trade name, manufactured by HIGUCHI, INC.), Bromelain (trade name, manufactured by GREAT FOOD CO., LTD. or GENENCOR KYOWA CO., LTD.), Papain W-40 (trade name, manufactured by AMANO ENZYME INC.), and food grade purified papain (manufactured by NAGASE CHEMTEX CORPORATION).

The animal milk casein to be used in step (A) or (B) to be discussed later is not particularly limited, as long as it is in the form of any food materials that contain animal milk casein having peptide sequences VPP, IPP, and YP, with functionality, such as antihypertensive activity. Examples of such food material may include animal milks, such as cow's milk, skim milk, processed milk, and animal milk casein extracts, optionally mixed with assistant agents for lactic acid bacteria fermentation, such as vitamins, nucleic acids, or yeast extracts.

In the animal milk casein to be used in the method of the present invention, the casein content is not particularly limited, and is usually about 3 to 15 wt%, preferably 3 to 10 wt%, for efficiently achieving the desired effects of the present invention.

In step (A), the amount of protease (1) to be used may suitably be decided for achieving the desired effects, depending on its titer. It is noted that, since step (A) of enzymatic digestion is performed in combination with step (B) of lactic acid bacteria fermentation to be discussed later, the amount of protease (1) for achieving the purpose may usually be smaller than that required for the enzymatic digestion of animal milk casein only with proteases. For example, the amount of protease (1) may preferably be 0.005 to 0.1 wt% of the amount of animal milk casein. At less than 0.005 wt%, the desired effects of the present invention may not be achieved, whereas at over 0.1 wt%, excess peptides are produced, which may impair the taste and flavor of the resulting product.

In step (A), the enzymatic digestion may be carried out at the optimum temperature and pH of protease (1), usually at 25 to 45 °C at around neutral pH. The time for the enzymatic digestion may suitably be decided depending on the amount and titer of protease (1).

When step (A) is to be performed prior to step (B) to be discussed later, after the enzymatic digestion, protease (1) may be inactivated at 60 to 100 °C, prior to step (B) . Alternatively, when steps (A) and (B) are to be performed simultaneously, i.e., when the animal milk casein is to be enzymatically digested and fermented with lactic acid bacteria at the same time in a single medium containing both protease (1) and the lactic acid bacteria, it is preferred to set the temperature to usually 25 to 45 °C, which is the optimum growth temperature for lactic acid bacteria, and the fermentation time to about 5 to 30 hours. The fermentation time may be shortened by adding a larger amount of the enzyme.

The method of the present invention includes step (B) of fermentation, with lactic acid bacteria, of animal milk casein and/or the protease (1) digestion product of animal milk casein.

The animal milk casein to be used in step (B) may be those mentioned above. When the animal milk casein is to be used in step (B), steps (A) and (B) are to be carried out simultaneously, as mentioned above. Alternatively, when the protease (1) digestion product is to be subjected to the fermentation with lactic acid bacteria in step (B), step (B) is to be carried out after step (A).

The lactic acid bacteria to be used in step (B) may be lactic acid bacteria of the genus *Streptococcus, Lactococcus, Lactobacillus, Bifidobacterium,* or the like, with *Lactobacillus* being preferred. More specific examples may include *Lactobacillus bulgaricus, Lactobacillus helveticus, Lactobacillus casei, Lactobacillus acidophilus,* and *Lactobacillus fermentum,* with *Lactobacillus helveticus, Lactobacillus acidophilus, Lactobacillus bulgaricus,* and *Lactobacillus casei* being preferred for their ability to produce VPP, IPP, and YP more effectively. One or more kinds of these lactic acid bacteria may be used.

The strain of *Lactobacillus helveticus* may preferably be those having a high extracellular proteinase activity. For example, strains having a U/OD590 value of not lower than 400 are preferred, as measured in accordance with the method of Yamamoto et al. (Yamamoto N. , et al., J. Biochem. (1993) 114, 740) based on the method of Twining et al. (Twining, S., Anal. Biochem. 143 3410 (1984).

An example of such preferred strains of *Lactobacillus helveticus* may be *Lactobacillus helveticus* CM4 (deposited at International Patent Organism Depositary, National Institute of Advanced Industrial Science and Technology, Tsukuba Central 6, 1-1-1 Higashi, Tsukuba-shi, Ibaraki, Japan, under accession number FERM BP-6060 on August 15, 1997) (referred to as CM4 hereinbelow). CM4 has been deposited under the above-mentioned accession number under the Budapest Treaty on the International Recognition of the Deposit of Microorganisms for the Purposes of Patent Procedure, and has already been patented.

The lactic acid bacteria are preferably in the form of a pre-cultured starter having sufficiently high activity. The initial cell count may preferably be about 10⁵-10⁹ cells/ml.

Step (B) may be performed as cofermentation with the lactic acid bacteria and a yeast for giving the resulting fermented milk improved taste, flavor, and the like. The strain of the yeast is not particularly limited, and may preferably be, for example, yeast of the genus *Saccharomyces,* such as *Saccharomyces cerevisiae.* The content of the yeast may suitably be selected for the purpose.

In the lactic acid bacteria fermentation, various assistant agents for lactic acid bacteria fermentation may additionally be used. Examples of such assistant agents may include amino acids, vitamins, minerals, nucleic acids, salts, microbial extracts, and detergents.

In step (B), the lactic acid bacteria fermentation may preferably be carried out usually at 25 to 45 °C, which is the optimum growth temperature of lactic acid bacteria, for 5 to 30 hours, either when step (B) is carried out simultaneously with step (A) or after step (A). Neutralizing fermentation is preferred for promoting fermentation.

According to the method of the present invention, a fermented milk may be obtained that contains any of VPP, IPP, and YP in an amount larger than that in a fermented milk obtained by ordinary lactic acid bacteria fermentation without step (A). Moreover, since the present method includes step (B), a product containing peptides, such as VPP, IPP, YP, or the like, may be obtained directly in the form of a fermented milk. Further, regardless of whether step (B) is carried out after step (A), or steps (A) and (B) are carried out simultaneously, at least one of VPP, IPP, and YP are expected to be produced in an amount larger than that produced merely by lactic acid bacteria fermentation.

The fermented milk food and beverages according to the present invention contain the fermented milk or a concentrate thereof, obtained by the above method of the present invention wherein an animal milk having a casein content of 3 to 10 wt% is used as the animal milk casein, and the amount of protease (1) is 0.005 to 0.1 wt% of the amount of casein, and contain not less than 1.2 mg/100 ml of VPP, not less than 1.0 mg/100 ml of IPP, and not less than 0.5 mg/100 ml of YP.

The contents of the particular peptides in the fermented milk food and beverages of the present invention may be measured by various HPLC. In particular, LC/MS method may be employed, which is one of the methods that facilitate convenient evaluation of various peptides. The LC/MS method reported by Matsuura et al. (Milchwissenschaft, 2004, 60, 24-27) may be used.

The fermented milk food and beverages according to the present invention are produced with the fermented milk obtained by the present method or a concentrate thereof, and may be in the form of, for example, yogurt, lactic acid bacteria beverages, lactic acid bacteria beverages made from dairy products, or various other processed milk food and beverages. The present fermented milk food and beverages may also be in the form of milk beverages for distribution at ordinary temperature or in chilled state, general food articles, dietary supplements, or the like.

The fermented milk food and beverages of the present invention may optionally contain various auxiliary additives for improving nutritional balance or taste and flavor, depending on its form. Examples of such auxiliary additives may include carbohydrates, lipids, vitamins, minerals, nutritional additives, sweeteners, flavoring agents, pigments, and texture improvers.

The fermented milk food and beverages according to the present invention may be provided as functional food and beverages, such as foods for specified health uses, or as functional food and beverages claiming antihypertensive effect, since the particular peptides, VPP, IPP, YP, and the like, known to produce antihypertensive effect are contained.

When the present fermented milk food and beverages are to be provided as functional food and beverages claiming antihypertensive effect, a preferred single intake is usually 0.1 ml/kg body weight to 5.0 ml/kg body weight, particularly 0. 3 ml/kg body weight to 0. 6 ml/kg body weight in terms of the fermented milk contained therein.

The present invention will now be explained in more detail with reference to Examples and Comparative Examples, which are illustrative only and do not intend to limit the present invention.

### Examples 1-3 and Comparative Examples 1-6

CM4 was pre-cultured at 37 °C for 20 hours in a milk medium composed of skim milk with a 9 wt% solid content which had been pasteurized at 100 °C, to prepare a lactic acid bacteria starter. Then 30 ml of a freshly prepared milk medium composed of skim milk with a 9 wt% solid content was inoculated with this starter at 3 wt%, and 0.5 mg of Papain F (trade name, manufactured by AMANO ENZYME INC., derived from Carica papaya L.) for Example 1, Purified Papain (trade name, manufactured by NAGASE BIOCHEMICALS, LTD., derived from Carica papaya L.) for Example 2, Bromelain F (trade name, manufactured by AMANO ENZYME INC., derived from Ananas comosus M.) for Example 3, Protease P (trade name, manufactured by AMANO ENZYME INC., derived from Aspergillus oryzae) for Comparative Example 1, Protease A (trade name, manufactured by AMANO ENZYME INC., derived from Aspergillus oryzae) for Comparative Example 2, Newrase F3G (trade name, manufactured by AMANO ENZYME INC. , derived from Rhizopus niveus) for Comparative Example 3, Neutrase (trade name, manufactured by NOVOZYMES, derived from Bacillus amyloliquefaciens) for Comparative Example 4, or Sumizyme FP (trade name, manufactured by SHIN NIHON CHEMICAL CO., LTD., derived from Aspergillus oryzae) for Comparative Example 5, was added thereto. For Comparative Example 6, a milk medium composed of the skim milk and inoculated with only the lactic acid bacteria starter without a protease was prepared. Each medium was incubated at 32 °C for 24 hours for enzymatic digestion and lactic acid bacteria fermentation, or lactic acid bacteria fermentation only, to prepare a fermented milk.

Each resulting fermented milk was centrifuged at 10000 g for 10 minutes, and the contents of VPP and IPP peptides in 1 ml of the culture supernatant were determined by LC/MS (manufactured by SHIMADZU CORPORATION, model LCMS2010A). The results are shown in Table 1.

### Comparative Examples 7-9

To 30 ml of a milk medium composed of skim milk with a 8 wt% solid content which had been pasteurized at 100 °C, was added 0.5 mg of Papain F (trade name, manufactured by AMANO ENZYME INC., derived from Carica papaya L.) for Comparative Example 7, which enzyme was used in Example 1, Purified Papain (trade name, manufactured by NAGASE BIOCHEMICALS, LTD., derived from Carica papaya L.) for Comparative Example 8, which enzyme was used in Example 2, or Bromelain F (trade name, manufactured by AMANO ENZYME INC., derived from Ananas comosus M.) for Comparative Example 9, which enzyme was used in Example 3. Each medium was incubated at 32 °C for 24 hours for enzymatic digestion to prepare an enzymatic digestion product. The contents of VPP and IPP peptides in each enzymatic digestion product were determined in the same way as in Example 1. The results are shown in Table 1.

Presence of peptides including a sequence Val-Pro-Pro or Ile-Pro-Pro was detected in the enzymatic digestion products prepared in Comparative Examples 7-9, and the sequences of the peptides were analyzed to identify Val-Pro-Pro-Phe-Leu and Ile-Pro-Pro-Leu-Thr in Comparative Examples 7 and 8, wherein the proteases identical with the ones used in Examples 1 and 2 were used, and Val-Pro-Pro-Phe-Leu-Gln-Pro-Glu-Val-Met and Ile-Pro-Pro-Leu-Thr in Comparative Example 9, wherein the protease identical with the one used in Example 3 was used.

From these results, it is assumed that the enzymatic digestion by the proteases in Examples 1 to 3 resulted in cleavage of the peptides represented by Xaa-Val-Pro-Pro-(Xbb)n and Xaa-Ile-Pro-Pro-(Xbb)n at the amino terminal side of the amino acid residue Xaa, while the lactic acid bacteria fermentation in Examples 1 to 3 resulted in cleavage of the same peptides at the carboxyl terminal side of the amino acid residue (Xbb)n, so that Val-Pro-Pro and Ile-Pro-Pro were efficiently produced in the resulting fermented milk.

**Table 1**

| | Trade name of protease | Lactic acid bacteria | VPP content (µg/ml) | IPP content (µg/ml) |
|---|---|---|---|---|
| Example 1 | Papain F | CM4 | 39 | 41 |
| Example 2 | Purified Papain | CM4 | 35 | 42 |
| Example 2 | Bromelain F | CM4 | 45 | 43 |
| Comp. Example 1 | Protease P | CM4 | 2 | 3 |
| Comp. Example 2 | Protease A | CM4 | 2 | 4 |
| Comp. Example 3 | Newrase F3G | CM4 | 5 | 3 |
| Comp. Example 4 | Neutrase | CM4 | 4 | 6 |
| Comp. Example 5 | Sumizyme | CM4 | 7 | 6 |
| Comp. Example 6 | - | CM4 | 26 | 18 |
| Comp. Example 7 | Papain F | - | 0 | 0 |
| Comp. Example 8 | Purified Papain | - | 0 | 0 |
| Comp. Example 9 | Bromelain F | - | 0 | 0 |

As shown in Table 1, in the fermentedmilk of Comparative Example 6 prepared only by the fermentation with lactic acid bacteria CM4, high productivity of VPP and IPP was confirmed. In the fermentedmilks of Examples 1 to 3 wherein the fermentation was combined with the use of the particular proteases including papain or bromelain, production of VPP and IPP in the amounts 1.7 to 1.9 times the amount in Comparative Example 1, was confirmed.

On the other hand, in the fermented milks of Comparative Examples 1 to 5 wherein the fermentation was combined with the use of proteases not including the particular proteases used in the present invention, the amounts of VPP and IPP produced were remarkably lower than those in the fermented milk of Comparative Example 6 prepared only by the fermentation with lactic acid bacteria CM4. Specifically, the amounts of VPP and IPP produced were remarkably lower even in the fermented milks of Comparative Examples 1, 2, and 5, wherein the proteases derived from Aspergillus were used, which are known to have the ability to produce VPP and IPP from animal milk casein. In the enzymatic digestion products of Comparative Examples 7 to 9, wherein only the proteases identical with those used in Examples 1 to 3 were used, no production of VPP and IPP was observed.

Thus the synergistic effect of the combination of the enzymatic digestion by the particular proteases and the lactic acid bacteria fermentation was confirmed.

### Example 4

Fermented milks were prepared in the same way as in Example 3, except that the amount of the protease, bromelain, was changed to 0.1 mg, 0.25 mg, and 1.0 mg, respectively, and the contents of VPP and IPP peptides in each fermented milk were determined in the same way as in Example 3. The results are shown in Fig. 1, which also shows the results of Example 3 wherein 0.5 mg of the protease, bromelain, was used, and of Comparative Example 6 wherein no protease was used. In Fig. 1, the left columns represent the VPP content, and the right columns represent the IPP content.

Here, 0.1 mg, 0.25 mg, 0.5 mg, and 1 mg of bromelain in 30 ml of the fermented milk correspond to the protease contents of 0.008 wt%, 0.021 wt%, 0.042 wt%, and 0.084 wt%, respectively, of the casein content in the fermented milk.

From Fig. 1, it is confirmed that addition of bromelain improved the productivity of the two peptides, compared to the case where bromelain was not added. In particular, it was confirmed that addition of the protease in the range of 0.25 to 1.0 mg resulted in improved VPP- and IPP-peptide productivity, which was about 1. 5 times the amount produced when the protease was not added.

### Example 5 and Comparative Example 10

CM4 was pre-cultured at 37 °C for 24 hours in a milk medium composed of skim milk with a 9 wt% solid content which had been pasteurized at 95 °C, to prepare a lactic acid bacteria starter. Then 1 liter of a freshly prepared milk medium composed of skim milk with a 9 wt% solid content was inoculated with this starter at 3 wt%, and 10 mg of Bromelain F (trade name, manufactured by AMANO ENZYME INC., derived from Ananas comosus M.) was added for Example 5. For Comparative Example 10, a milk medium composed of the skim milk and inoculated with only the lactic acid bacteria starter without a protease was prepared. Each medium was incubated at 32 °C for 20 hours for enzymatic digestion and lactic acid bacteria fermentation, or lactic acid bacteria fermentation only, to prepare a fermented milk.

To each resulting fermented milk, a stabilizer and flavoring agents were added, and ultimately 120 g each of fermented milk drinks having a fermented milk content of about 60 wt% and 30 wt% were prepared. The contents of VPP, IPP, and YP in each fermented milk drink were determined in the same way as in Example 1. The acidity of each fermented milk was also measured. The results are shown in Table 2.

**Table 2**

| | Content of fermented milk (wt%) | VPP content (µg/ml) | IPP content (µg/ml) | YP content (µg/ml) | Acidity (%) |
|---|---|---|---|---|---|
| Example 5 | 60 | 36 | 34 | 20 | 1.3 |
| | 30 | 19 | 17 | 9.6 | 0.6 |
| Comp.Ex. 10 | 60 | 19 | 12 | 7.2 | 1.3 |
| | 30 | 9.5 | 6.1 | 3.7 | 0.7 |

From Table 2, it is understood that in the fermented milk drink with about 60 wt% fermented milk of Comparative Example 10, which was prepared only by fermentation with lactic acid bacteria CM4, the total content of the antihypertensive peptides, VPP and IPP, was 31 µg/ml, so that 120 g of the drink contained in total of 3.7 mg of the two tripeptides.

On the other hand, it is also understood that, in the fermented milk drink with about 60 wt% fermented milk of Example 5, which was fermented with bromelain added, 8.4 mg of the tripeptides were contained in 120 g of the drink, according to the same calculation.

Further, according to the same calculation, in the fermented milk drink with about 30 wt% fermented milk of Comparative Example 10, 1.9 mg of the tripeptides were contained in 120 g of the drink, whereas in the fermented milk drink with about 30 wt% fermented milk of Example 5, 4.3 mg of the tripeptides were contained in 120 g of the drink. Production of YP was also observed, which has antihypertensive activity confirmed in animal tests.

It is known that the minimum effective dose per intake of VPP and IPP peptides together for achieving the antihypertensive effect in human is not less than 3.4 mg in terms of VPP (VPP + 1.7 x IPP). Thus, for providing the fermented milk drink with about 60 wt% fermented milk of Comparative Example 10 as functional food claiming the antihypertensive effect, the drink must be made into a 120g product. In contrast, the fermented milk drink with about 60 wt% fermented milk of Example 5 satisfies the above minimum effective dose even in about a 60g product, and the fermented milk drink with about 30 wt% fermented milk of Example 5, when made into a 120g product, satisfies the above minimum effective dose even though the fermented milk content thereof is half the amount of the 60 wt% drink of Comparative Example 10. Further, as a result of evaluation of taste and flavor of the fermented milks per se prepared in Example 5 and Comparative Example 10, no remarkable difference was observed in taste and flavor between the fermented milks with and without the protease.

From the results discussed above, it is understood that the fermented milk drink containing the fermented milk obtained by the method of the present invention may be provided as functional food that claims the antihypertensive effect, even at a smaller content of the fermented milk, compared to the amount of the conventional CM4-fermented milk required. Thus freedom in blending for designing taste and flavor of the final product is increased, which allows manufacture of products having high quality taste and flavor.

### Referential Example 1

In order to determine whether lactic acid bacteria other than *Lactobacillus helveticus* could produce Val-Pro-Pro and Ile-Pro-Pro from the peptides, Val-Pro-Pro-Phe-Leu and Ile-Pro-Pro-Leu-Thr, which were detected in Examples 1 and 2 and Comparative Examples 7 and 8, the amounts of Val-Pro-Pro and Ile-Pro-Pro produced by culture of a lactic acid bacteria medium containing the two pentapeptides, were measured. As the lactic acid bacteria, *Lactobacillus bulgaricus* JCM1002 strain, *Lactobacillus acidophilus* JCM1132 strain, and *Lactobacillus casei ssp. casei* JCM1136 strain (all at Microbe Division, Japan Collection of Microorganisms, RIKEN BioResource Center, 2-1 Hirosawa, Wako, Saitama, Japan) were used.

10 µg/ml each of chemically synthesized Ile-Pro-Pro-Leu-Thr and Val-Pro-Pro-Phe-Leu were added to a MRS medium, which is a lactic acid bacteria medium, and incubated with each strain at 37 °C for 4 days. The concentrations of Val-Pro-Pro and Ile-Pro-Pro in the supernatant fraction were measured by LC/MS. The results are shown in Table 3.

**Table 3**

| | VPP content (µg/ml) | IPP content (µg/ml) |
|---|---|---|
| *Lactobacillus helveticus* CM4 strain | 1.1 | 0.3 |
| *Lactobacillus bulgaricus* JCM1002 strain | 2.2 | 0.6 |
| *Lactobacillus acidophilus* JCM1132 strain | 5.2 | 3.2 |
| *Lactobacillus casei* JCM1136 strain | 7.6 | 4.4 |

The results of Referential Example 1 show that all of the bacterial strains produced Val-Pro-Pro and Ile-Pro-Pro. It is expected that VPP and IPP were contained in the fermented milks prepared above by lactic acid bacteria fermentation in combination with the proteases that produce Val-Pro-Pro-Phe-Leu and Ile-Pro-Pro-Leu-Thr from casein. Thus it is understood that not only *Lactobacillus helveticus* but also other lactic acid bacteria may be used.

## Claims

1. A method for producing a fermented milk comprising the steps of:
(A) enzymatically digesting animal milk casein with protease (1) comprising at least one of papain, bromelain, and proteases belonging to a superfamily that shows enzyme reaction similar to these, and
(B) fermenting, with lactic acid bacteria, animal milk casein and/or protease (1) -digestion product of animal milk casein,
to thereby produce at least one of Val-Pro-Pro, Ile-Pro-Pro, and Tyr-Pro in a resulting fermented milk.

2. The method according to claim 1, wherein said steps (A) and (B) are carried out in a medium containing both said protease (1) and said lactic acid bacteria to enzymatically digest and ferment said animal milk casein.

3. The method according to claim 1, wherein said protease (1) comprises a protease that cleaves at least one peptide represented by (Xaa)m-Val-Pro-Pro-(Xbb)n, (Xaa)m-Ile-Pro-Pro-(Xbb)n, or (Xaa)m-Tyr-Pro-(Xbb)n at the carboxyl terminal side of (Xaa)m, wherein Xaa and Xbb each independently stands for an arbitrary amino acid; Xaa in one peptide sequence may be the same as or different from Xaa in the other peptide sequences; Xbb in one peptide sequence may be the same as or different from Xbb in the other peptide sequences; m and n are integers; when m is 2 or more, Xaa's may be the same or different; and when n is 2 or more, Xbb's may be the same or different.

4. The method according to claim 1, wherein said protease (1) comprises a protease belonging to a subfamily of papain.

5. The method according to claim 1, wherein said protease (1) comprises a protease contained in a pineapple extract and/or a papaya extract.

6. The method according to claim 1, wherein said lactic acid bacteria comprise at least one of *Lactobacillus helveticus, Lactobacillus acidophilus, Lactobacillus bulgaricus,* and *Lactobacillus casei.*

7. The method according to claim 1, wherein said enzymatic digestion and lactic acid bacteria fermentation in steps (A) and (B) are carried out at 25 to 45 °C.

8. The method according to claim 1, wherein an animal milk having a casein content of 3 to 10 wt% is used as said animal milk casein, and an amount of said protease (1) added is 0.005 to 0.1 wt% of the amount of said casein.

9. Fermented milk food and beverages comprising a fermented milk obtained by the method of claim 8 or a concentrate thereof, and containing not less than 1.2 mg/100 ml of Val-Pro-Pro, not less than 1.0 mg/100 ml of Ile-Pro-Pro, and not less than 0.5 mg/100 ml of Tyr-Pro.
